# EUROPEAN PATENT APPLICATION

(11) **EP 2 939 666 A1**
(43) Date of publication of application: **04.11.2015**
(21) Application number: 15165197.3
(22) Date of filing: 27.04.2015
(51) Int. Cl.: A61K 9/20, A61K 31/192, A61K 9/16

(54) **PHARMACEUTICAL FORMULATIONS OF LOXOPROFEN**

(30) Priority: 29.04.2014 TR 201404790
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Erdem, Yelda, 34460 Istanbul (TR); Ataman, Seval, 34460 Istanbul (TR)
(74) Representative: Sevinç, Erkan

(57) **Abstract**

The present invention relates to pharmaceutical formulation comprising loxoprofen or a pharmaceutically acceptable salt thereof and one or more pharmaceutically acceptable excipients.

## Description

### Technical Field of the Invention

The present invention relates to pharmaceutical formulation comprising loxoprofen or a pharmaceutically acceptable salt thereof and one or more pharmaceutically acceptable excipients.

### Background of the Invention

Loxoprofen is a non-steroidal anti-inflammatory drug in the propionic acid derivatives group. It is a prodrug and it is quickly converted to its active trans-alcohol metabolite following oral administration. It is a non-selective cyclooxygenase inhibitor and works by reducing the synthesis of prostaglandins from arachidonic acid. Its chemical name is (RS)-2-{4-[(2-oxocyclopentyl)methyl]phenyl}propanoic acid and its chemical structure is shown in the Formula I.

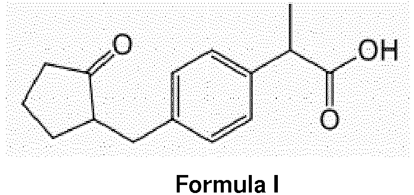

The patent application US4161538 (A) discloses the loxoprofen molecule.

The patent JP2669517 (B2) discloses a solid preparation containing loxoprofen sodium and additives such as microcrystalline cellulose, hydroxypropyl cellulose having low substitution degree, lactose or magnesium stearate, in the form of a tablet, capsule, granule or powder.

The patent EP0947584 (B1) discloses an anti-inflammatory analgesic patch comprising loxoprofen or pharmaceutically acceptable salt thereof, water, crotamiton and a water soluble polymer.

The patent application WO0247661 (A1) discloses pharmaceutical composition for intramuscular injection containing loxoprofen or a pharmaceutically acceptable salt thereof, as an active ingredient.

The patent application JP2010270140 (A) discloses an oral pharmaceutical composition which alleviates gastric mucosa disorders caused by loxoprofen, comprises one or more sugars and sugar alcohols selected from the group consisting of sucrose, maltitol, fructose, xylitol, trehalose, lactose and lactitol; and loxoprofen. A tablet, subtle granule (powder is included), capsule, solution (syrup is included), etc., may be cited as a concrete dosage form of the oral pharmaceutical composition.

The patent EP1806152 (B1) discloses an external preparation containing a pharmacologically active component that is loxoprofen and a lipophilic polyglycerin fatty acid ester.

In the state of art, there are several patents and applications which disclose many formulations of loxoprofen or a pharmaceutically acceptable salt thereof, in different dosage forms. In the present invention a novel tablet formulation of loxoprofen or a pharmaceutically acceptable salt thereof is disclosed. The aims of this invention are to obtain favorable uniformity and homogeneity of the loxoprofen tablet providing higher bioavailability while eliminating the undesirable side effects of the drug and to get improved flow properties of dry powders as tableting. Also, with this invention, stability, mechanical strength (such as; hardness and friability) and compressibility of the tablet formulation are improved by using appropriate excipients.

### Detailed Description of the Invention

The present invention relates to pharmaceutical formulation comprising loxoprofen or a pharmaceutically acceptable salt thereof and one or more pharmaceutically acceptable excipients.

According to one embodiment, pharmaceutically acceptable salt of loxoprofen is sodium hydrate.

According to one embodiment, loxoprofen sodium hydrate is present in an amount of 10 to 40 % and preferably 20 to 30 % by weight of total formulation.

The formulation according to the present invention is in the form of a tablet.

The pharmaceutical tablet formulations according to the present invention may also comprise one or more pharmaceutically acceptable excipient(s). Pharmaceutically acceptable excipients comprise, but are not limited to disintegrants, fillers, binders, lubricants, glidants or the mixtures thereof.

In a preferred embodiment of the present invention, said disintegrants comprise, but are not limited to low-substituted hydroxypropyl cellulose, hydroxypropyl cellulose, alginic acid and alginates, ion-exchange resins, magnesium aluminum silicate, sodium dodecyl sulphate, sodium carboxy methyl cellulose, carboxy methyl cellulose calcium, polyvinylpyrrolidone, docusate sodium, guar gam, polyacrylin potasium, poloxomer, sodium alginate, sodium glysin carbonate, sodium lauryl sulphate or the mixtures thereof, preferably, it is a grade of low-substituted hydroxypropyl cellulose which is L-HPC (LH21).

It is known that obtaining an adequate content uniformity is an important issue for pharmaceutical solid dosage forms during the process, because it is difficult to disintegrate active ingredients homogenously. Moreover, inadequate content uniformity results undesired side effects and low bioavailability during the therapy. In this invention, L-HPC (LH21) is used as a disintegrant in order to obtain desirable content uniformity. Low-substituted hydroxypropyl cellulose is a stable material that is widely used in oral solid-dosage forms and especially L-HPC (LH21) that is less fibrous is appropriate disintegrant for tablets through the wet-granulation process. The present invention offers a loxoprofen tablet formulation with more favorable homogeneous and uniform content.

According to one embodiment, L-HPC (LH21) is present in an amount of 2 to 40 % and preferably 5 to 20 % by weight of total formulation.

In a preferred embodiment of the present invention, said glidants comprise, but are not limited to colloidal silicon dioxide, stearic acid, talc, aluminium silicate or the mixtures thereof, preferably, it is colloidal silicon dioxide.

Colloidal silicon dioxide is widely used in pharmaceuticals, cosmetics, and food products. Its small particle size and large specific surface area give it desirable flow characteristics that are exploited to improve the flow properties of dry powders in a number of processes such as tableting and capsule filling.

In a preferred embodiment of the present invention, it has surprisingly been found that the use of L-HPC (LH21) and colloidal silicon dioxide together make better contribution to desirable uniformity and homogeneity of the loxoprofen tablet providing higher bioavailability while eliminating the undesirable side effects of the drug. Moreover, the lactose monohydrate also allows better mixing with other formulation ingredients.

According to one embodiment, colloidal silicon dioxide is present in an amount of 0.1 to 1 % and preferably 0.5 to 1 % by weight of total formulation.

In a preferred embodiment of the present invention, said fillers comprise, but are not limited to lactose monohydrate, dibasic calcium phosphate, tribasic calcium phosphate, sorbitol, sucrose, trehalose, isomalt, microcrystalline cellulose, mannitol, starch, sodium carbonate, sodium bicarbonate, dextrose, maltodextrine, calcium carbonate, xylitol or the mixtures thereof, preferably, it is lactose monohydrate.

According to one embodiment, lactose monohydrate is present in an amount of 30 to 60 % and preferably 45 to 55 % by weight of total formulation.

In a preferred embodiment of the present invention, said binders comprise, but are not limited to hydroxypropyl cellulose, sugars, glucose syrups, natural gums, guar gum, gelatins, pullulan, polymetacrylates, collagen, agar, algynate, sodium alginate, hyaluronic acid, pectin, tragacanth gum, carboxymethyl cellulose, polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide or the mixtures thereof, preferably, it is a grade of hydroxypropyl cellulose which is HPC-LF.

In a preferred embodiment of the present invention, it has surprisingly been found that the combination of HPC-LF and L-HPC (LH21) creates a synergistic effect over stability, mechanical strength (such as; hardness and friability) and compressibility of the tablet formulation.

According to one embodiment, HPC-LF is present in an amount of 1 to 10 % and preferably 5 to 10 % by weight of total formulation. Besides, the viscosity of HPC-LF is between 75.0 % and 150.0 % cp (at 25°C).

The viscosity of HPC-LF is measured by the following method;

While stirring, introduce a quantity of the substance to be examined equivalent to 15.00 g of the dried substance into 150 g of water heated to 90 °C. Stir with a propeller-type stirrer for 10 min, place the flask in a bath of iced water, continue the stirring and allow to remain in the bath of iced water for 40 min to ensure that dissolution is complete. Adjust the mass of the solution to 300 g and centrifuge the solution to expel any entrapped air. Determine the viscosity with a rotating viscometer at 25 °C.

In a preferred embodiment of the present invention, said lubricants comprise, but are not limited to magnesium stearate, sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin or the mixtures thereof, preferably, it is magnesium stearate.

According to one embodiment, magnesium stearate is present in an amount of 0.25 to 5 % and preferably 0.5 to 2 % by weight of total formulation.

In this present invention, the formulation has been designed comprising the following:
a) 20 to 30 % by weight of loxoprofen sodium hydrate
b) 45 to 55 % by weight of lactose monohydrate
c) 5 to 10 % by weight of HPC-LF
d) 5 to 20 % by weight of L-HPC (LH-21)
e) 0.5 to 2 % by weight of magnesium stearate
f) 0.5 to 1 % by weight of colloidal silicon dioxide

**Inner phase:** Inner phase comprises loxoprofen sodium hydrate, lactose monohydrate, HPC-LF and 2/3 of L-HPC (LH-21) by weight.

**Outer phase:** Outer phase comprises 1/3 of L-HPC (LH-21) by weight, colloidal silicon dioxide and magnesium stearate.

The tablet formulation according to the present invention is produced by wet granulation.

In this present invention, the wet granulation method comprising the following steps;
a) Loxoprofen sodium hydrate, lactose monohydrate, HPC-LF and 2/3 of L-HPC (LH-21) by weight, are sieved and mixed and homogeneous mixture is obtained.
b) After obtaining a homogeneous mixture, wet granulation is made with water.
c) The obtained granules in part (b) are dryed in an oven till a predetermined water content is obtained.
d) The obtained granules in part (c) are sieved.
e) The sieved granules in part (d) are mixed with the remaining part of L-HPC (LH-21) by weight.
f) The mixture in part (e) is mixed with colloidal silicon dioxide.
g) The mixture in part (f) is mixed with magnesium stearate.
h) The final mixture is compressed as tablets.

### Example 1 - Tablet formulation of loxoprofen

| **Ingredients** | **Amount (%)** |
|---|---|
| Loxoprofen sodium hydrate | 10-40 |
| Lactose monohydrate | 30-60 |
| HPC-LF | 1-10 |
| L-HPC (LH-21) | 2-40 |
| Magnesium stearate | 0.25-5 |
| Colloidal silicon dioxide | 0.1 - 1 |

The pharmaceutical formulation mentioned above is prepared as following:
Loxoprofen sodium hydrate, lactose monohydrate, HPC-LF and 2/3 of L-HPC (LH-21) by weight, are sieved and mixed. After obtaining a homogeneous mixture, wet granulation is made with water and dryed in an oven till a predetermined water content is obtained. After the obtained granules are sieved they mixed with the remaining part of L-HPC (LH-21) by weight, and mixed with colloidal silicon dioxide, and then mixed with magnesium stearate and compressed as tablets.

## Claims

1. A pharmaceutical formulation comprising loxoprofen or a pharmaceutically acceptable salt thereof and one or more pharmaceutically acceptable excipients.

2. The pharmaceutical formulation according to claim 1, wherein pharmaceutically acceptable salt of loxoprofen is sodium hydrate.

3. The pharmaceutical formulation according to claim 1 or 2, wherein loxoprofen sodium hydrate is present in an amount of 10 to 40 % and preferably 20 to 30 % by weight of total formulation.

4. The pharmaceutical formulation according to claim 1 to 3, wherein the pharmaceutical formulation is in the form of a tablet.

5. The pharmaceutical tablet formulation according to claim 1 to 4, wherein one or more pharmaceutically acceptable excipient is selected from the group comprising disintegrants, fillers, binders, lubricants, glidants or the mixtures thereof.

6. The pharmaceutical tablet formulation according to claim 5, wherein the disintegrant is selected from the group comprising low-substituted hydroxypropyl cellulose, hydroxypropyl cellulose, alginic acid and alginates, ion-exchange resins, magnesium aluminum silicate, sodium dodecyl sulphate, sodium carboxy methyl cellulose, carboxy methyl cellulose calcium, polyvinylpyrrolidone, docusate sodium, guar gam, polyacrylin potasium, poloxomer, sodium alginate, sodium glysin carbonate, sodium lauryl sulphate or the mixtures thereof, preferably, it is a grade of low-substituted hydroxypropyl cellulose which is L-HPC (LH21).

7. The pharmaceutical tablet formulation according to claim 6, wherein L-HPC (LH21) is present in an amount of 2 to 40 % and preferably 5 to 20 % by weight of total formulation.

8. The pharmaceutical tablet formulation according to claim 5, wherein the glidant is selected from the group comprising colloidal silicon dioxide, stearic acid, talc, aluminium silicate or the mixtures thereof, preferably, it is colloidal silicon dioxide.

9. The pharmaceutical tablet formulation according to claim 8, wherein colloidal silicon dioxide is present in an amount of 0.1 to 1 % and preferably 0.5 to 1 % by weight of total formulation.

10. The pharmaceutical tablet formulation according to claim 5, wherein the filler is selected from the group comprising lactose monohydrate, dibasic calcium phosphate, tribasic calcium phosphate, sorbitol, sucrose, trehalose, isomalt, microcrystalline cellulose, mannitol, starch, sodium carbonate, sodium bicarbonate, dextrose, maltodextrine, calcium carbonate, xylitol or the mixtures thereof, preferably, it is lactose monohydrate.

11. The pharmaceutical tablet formulation according to claim 10, wherein lactose monohydrate is present in an amount of 30 to 60 % and preferably 45 to 55 % by weight of total formulation.

12. The pharmaceutical tablet formulation according to claim 5, wherein the binder is selected from the group comprising hydroxypropyl cellulose, sugars, glucose syrups, natural gums, guar gum, gelatins, pullulan, polymetacrylates, collagen, agar, algynate, sodium alginate, hyaluronic acid, pectin, tragacanth gum, carboxymethyl cellulose, polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide or the mixtures thereof, preferably, it is a grade of hydroxypropyl cellulose which is HPC-LF.

13. The pharmaceutical tablet formulation according to claim 12, wherein HPC-LF is present in an amount of 1 to 10 % and preferably 5 to 10 % by weight of total formulation.

14. The pharmaceutical tablet formulation according to claims 12 or 13, wherein viscosity of HPC-LF is between 75.0 % and 150.0 % cp (at 25°C).

15. The pharmaceutical tablet formulation according to claim 5, wherein the lubricant is selected from the group comprising magnesium stearate, sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin or the mixtures thereof, preferably, it is magnesium stearate.

16. The pharmaceutical tablet formulation according to claim 15, wherein magnesium stearate is present in an amount of 0.25 to 5 % and preferably 0.5 to 2 % by weight of total formulation.

17. The pharmaceutical tablet formulation according to any of the preceding claims comprising;
a) 20 to 30 % by weight of loxoprofen sodium hydrate
b) 45 to 55 % by weight of lactose monohydrate
c) 5 to 10 % by weight of HPC-LF
d) 5 to 20 % by weight of L-HPC (LH-21)
e) 0.5 to 2 % by weight of magnesium stearate
f) 0.5 to 1 % by weight of colloidal silicon dioxide

18. The pharmaceutical tablet formulation according to any of the preceding claims, wherein the pharmaceutical tablet formulation is produced by wet granulation.

19. The pharmaceutical tablet formulation according to any of the preceding claims, wherein the wet granulation method comprising the following steps;
a) Loxoprofen sodium hydrate, lactose monohydrate, HPC-LF and 2/3 of L-HPC (LH-21) by weight, are sieved and mixed and homogeneous mixture is obtained.
b) After obtaining a homogeneous mixture, wet granulation is made with water.
c) The obtained granules in part (b) are dryed in an oven till a predetermined water content is obtained.
d) The obtained granules in part (c) are sieved.
e) The sieved granules in part (d) are mixed with the remaining part of L-HPC (LH-21) by weight.
f) The mixture in part (e) is mixed with colloidal silicon dioxide.
g) The mixture in part (f) is mixed with magnesium stearate.
h) The final mixture is compressed as tablets.
